# FASCICULE DE BREVET EUROPEEN

(11) **EP 0 461 045 B1**
(45) Date de publication et mention de la délivrance du brevet: **11.09.1996**
(21) Numéro de dépôt: 91401501.1
(22) Date de dépôt: 07.06.1991
(51) Int. Cl.: C12Q 1/68, C12P 19/34, C07H 21/04, C12N 15/11

(54) **Détection spécifique du mycobacterium tuberculosis**
Spezifischer Nachweis von Mycobacterium Tuberculosis
Specific detection of mycobacterium tuberculosis

(30) Priorité: 08.06.1990 FR 9007192
(43) Date de publication de la demande: 11.12.1991
(73) Titulaire: INSTITUT PASTEUR, F-75724 Paris Cédex 15 (FR)
(72) Inventeur: Guesdon, Jean-Luc, F-92310 Sévres (FR); Thierry, Dominique, F-92100 Boulogne (FR)
(74) Mandataire: Desaix, Anne

(56) Documents cités:
- WO-A-90/10085
- FR-A- 2 651 505
- NUCLEIC ACIDS RESEARCH vol.18, no.1, 11 janvier 1990, page 188, Oxford University Press; D.Thierry et al: "IS6110, an IS-like element of Mycobacterium tuberculosis complex"
- JOURNAL OF GENERAL MICROBIOLOGY vol.135, no.9, septembre 1989, pages 2347-2355, Society for General Microbiology; Z.F.Zainuddin et al: "Polymorphic repetitive DNA sequences in Mycobacterium tuberculosis detected with a gene probe from a Mycobacterium fortuitem plasmid"
- JOURNAL OF CLINICAL MICROBIOLOGY vol.28, no.3, mars 1990, pages 513-518, American Society for Microbiology; R.J.Patel et al: "Sequence analysis and amplification by polymerase chain reaction of a cloned DNA fragment for identification of Mycobacterium tuberculosis"
- INTERNATIONAL JOURNAL OF LEPROSY vol.56, no.4, décembre 1988, pages 592-598, US; P.P.Reddi et al: "Repetitive DNA sequence to Mycobacterium tuberculosis: analysis of differential hybridization pattern with other mycobacteria"
- MOLECULAR CLONING: A LABORATORY MANUAL; Second Edition J. Sambrook et al; Coldspring Habor. Laborating Press; Pages; 1.11; 1.13 and 1.13.
- T198/84; Proc.Natl.Acad.Sci. USA 1986, vol.83, pages 5939-5942

## Description

L'invention concerne une séquence d'acide nucléique spécifique du Mycobacterium tuberculosis, ainsi que des fragments particuliers de cette séquence aptes à jouer le rôle d'amorces nucléiques dans l'amplification d'ADN provenant de Mycobacterium dans un échantillon biologique. L'invention concerne également une méthode de détection de Mycobacterium tuberculosis dans un échantillon biologique, cette méthode mettant en oeuvre lesdites amorces nucléiques.

Les mycobactéries correspondent au genre Mycobaccerium qui comprend au moins 54 espèces différentes.

Parmi celles-ci, environ 10 sont pathogènes ou opportunistes pour l'homme ou l'animal. M. tuberculosis est l'agent de la tuberculose.

Il est connu que cette maladie représente un problème majeur de Santé Publique ; en effet, il existe actuellement entre 15 et 60 millions d'individus atteints; de tuberculose dans le monde et 2 à 3 millions de personnes meurent chaque année à cause de cette infection. Dans les pays développés, M. tuberculosis est la cause la plus commune des infections mycobactériennes. En France, il apparaît environ 10⁴ nouveaux cas de tuberculose par an. La vaccination par le BCG (Bacille de Calmette et Guérin, une souche atténuée de M. bovis) est loin d'être efficace au sein de toutes les populations. Cette efficacité varie environ de 80 % dans les pays occidentaux comme l'Angleterre à 0 % en Inde (résultats du dernier essai de vaccination à Chingleput). De plus, l'apparition de souches de M. tuberculosis résistantes aux antituberculeux usuels et l'existence d'une corrélation entre tuberculose et SIDA ajoute à l'urgence de mettre au point une méthode rapide de détection et d'identification des mycobactéries.

Par exemple, une étude épidémiologique réalisée en Floride a montré que 10 % des malades atteints de SIDA sont atteints de tuberculose au moment du diagnostic du SIDA ou 18 mois avant celui-ci. Chez ces malades, la tuberculose apparaît dans 60 % des cas sous une forme disséminée donc non repérable par les critères de diagnostic classiques comme la radiographie pulmonaire ou l'analyse de crachats.

Enfin, le diagnostic de la tuberculose et des autres mycobactérioses apparentées est difficile à réaliser pour différentes raisons : les maladies pulmonaires causées par différentes mycobactéries ne peuvent pas être distinguées cliniquement, radiologiquement ou histologiquement ; les mycobactéries sont souvent présentes en faible quantité et lorsqu'elles sont en quantité détectable par les méthodes classiquement utilisées, la maladie est déjà en évolution et les malades sont contagieux pour leur entourage ; de plus, en raison du temps de génération très long de ces bactéries (24 h pour M. tuberculosis comparé à 20 min pour E. coli), la culture de ces organismes est difficile. Ainsi faut-il 6 à 8 semaines pour identifier les germes et davantage pour obtenir un antibiograme utilisable pour le traitement adéquat des malades. La nécessité d'un test de détection n'exigeant pas de culture des germes et directement utilisable avec les échantillons pathologiques, même lorsque les germes y sont présents à de faibles concentrations, est donc indispensable.

Plusieurs techniques sont actuellement utilisées en clinique pour identifier une infection mycobactérienne.

Il faut tout d'abord citer la détection directe des microorganismes au microscope ; cette technique est rapide mais ne permet pas l'identification de l'espèce mycobactérienne observée et manque de sensibilité dans la mesure où un grand nombre de microorganismes doit être présent dans l'échantillon (>10⁴/ml) pour permettre une détection fiable (BATES J., CHEST, 1979, 76, (suppl.), 757-763).

Les cultures, lorsqu'elles sont positives, ont une spécificité approchant 100 % et permettent l'identification de l'espèce mycobactérienne isolée ; néanmoins, comme précisé ci-dessus, la croissance des mycobactéries in vitro ne peut être réalisée qu'en 3 à 6 semaines et lorsque peu de mycobactéries sont présentes au site de l'infection, des cultures répétées sont nécessaires pour s'assurer d'un résultat positif (BATES J., 1979 et BATES J. et al., Am. Rev. Respir. Dis., 1986, 134, 415-417).

Les techniques sérologiques peuvent s'avérer utiles dans certaines conditions mais leur utilisation est limitée par leur sensibilité et/ou leur spécificité faibles (DANIEL T.M. et al., Am. Rev. Respir. Dis., 1987, 135, 1137-1151).

La présence ou l'absence de mycobactéries peut également être déterminée par hybridation avec de l'ADN ou de l'ARN en utilisant des sondes spécifiques des séquences d'ADN (KIEHN T.E. et al., J. Clin. MIcrobiol., 1987, 25, 1551-1552 ; ROBERTS M.C. et al., J. Clin. Microbiol., 1987, 25, 1239-1243 ; DRAKE T.A. et al., J. Clin. Microbiol., 1987, 25, 1442-1445). Cependant, ces méthodes reposent sur le polyomorphisme des séquences nucléotidiques des fragments utilisés ou sur le polymorphisme des régions avoisinantes et nécessitent également la culture des microorganismes.

THIERRY et al. (Nucl. Acid Res., Vol. 18 n° 1, p 188) ont décrit une séquence spécifique du complexe Mycobacterium tuberculosis et nommée IS 6110. Les auteurs proposent d'utiliser cette séquence comme sonde nucléique dans la détection de Mycobacterium tuberculosis.

Cependant, les quantités d'ADN mycobactérien présentes dans la plupart des échantillons biologiques sont insuffisantes pour donner un signal positif ; la technique d'hybridation par sonde nucléique s'est donc révélée inadaptée à l'identification d'ADN mycobactérien extrait directement d'échantillons biologiques.

Certains auteurs ont proposé, pour surmonter ce problème, d'amplifier spécifiquement l'ADN provenant du mycobactérium en utilisant des amorces nucléiques dans une méthode d'amplification telle que la réaction de polymérase en chaîne (P.C.R.). PATEL et al. (J. Clin., Microbiol., Mar. 1990, 513-518) ont décrit l'utilisation de plusieurs amorces nucléiques choisies à partir d'une séquence connue en tant que sonde dans l'identification de M. tuberculosis. Cependant, la longueur des fragments obtenue en utilisant ces amorces était différente de la longueur théoriçue attendue, et plusieurs fragments de taille variable étaient obtenus. De plus, les auteurs ont observé l'absence d'hybridation des produits amplifiés avec le plasmide ayant servi à déterminer les amorces. Ces résultats indiquent que ces amorces ne seraient pas appropriées dans la détection de la présence de M. tuberculosis dans un échantillon biologique et confirment la nature critique du choix des amorces.

L'objet de la présente invention est de fournir une méthode de détection de M. tuberculosis qui est à la fois spécifique, sensible et fiable, et qui ne nécessite pas de culture préalable des mycobactéries. L'invention concerne un fragment d'acide nucléique dérivé du génome de Mycobacterium tuberculosis caractérisé en ce qu'il est constitué d'une des séquences I, II, III et IV, définie de la façon suivante :
I : une séquence choisie parmi l'une des séquences A à H : 
A : 5' - CCCGCGGCAAAGCCCGCAGGACCACGATCG - 3'
B : 5' - CGACCCGCCAGCCCAGGATCCTGCGAGCGT - 3'
C : 5' - GGCGGGTCCAGATGGCTTGCTCGATCGCGT - 3'
D : 5' - GTTGGCGGGTCCAGATGGCTTGCTCGATCG - 3'
E : 5' - TCAAAGGGTTTGACAAATTAATGATTGGTC - 3'
F : 5' - TCGTGTACAAAATGTGGACAAGTA - 3'
G : 5' - TCGACGGACGTCGTGACCAGAAGTC - 3'
H : 5' - GTCGACACGCCTTCTGCACGGAAGTCCTT - 3'
II : une séquence comportant au moins 10 bases consécutives de l'une des séquences A à H et ayant une longueur totale d'environ 20 à 40 bases ;
III : une séquence ayant une longueur de 20 à 40 bases qui hybride avec la séquence I ou avec la séquence II, et qui présente de préférence au moins 80 % d'homologie avec celles-ci ;
IV : une séquence complémentaire à l'une des séquences I, II ou III.

L'invention concerne également un couple de fragments d'acide nucléique dérivés du génome de Mycobacterium tuberculosis et aptes à jouer le rôle d'amorces nucléiques dans l'amplification de l'ADN provenant dudit Mycobacterium dans un échantillon biologique, caractérisé en ce qu'il est constitué de deux séquences sélectionnées parmi les séquences I à IV selon la revendication 1.

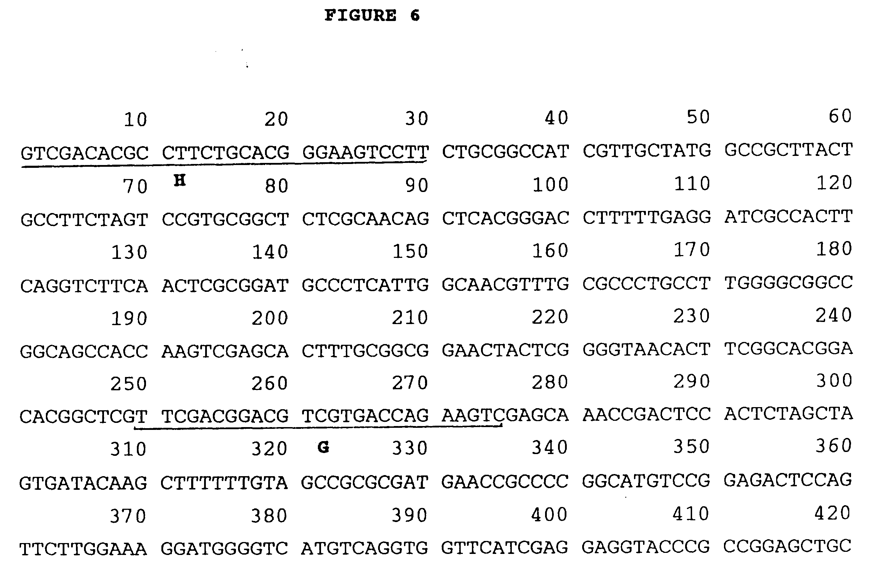

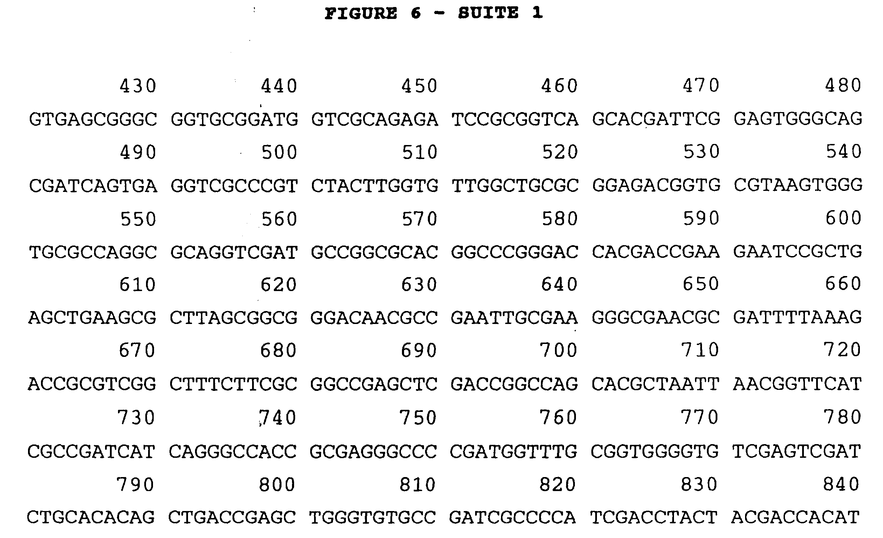

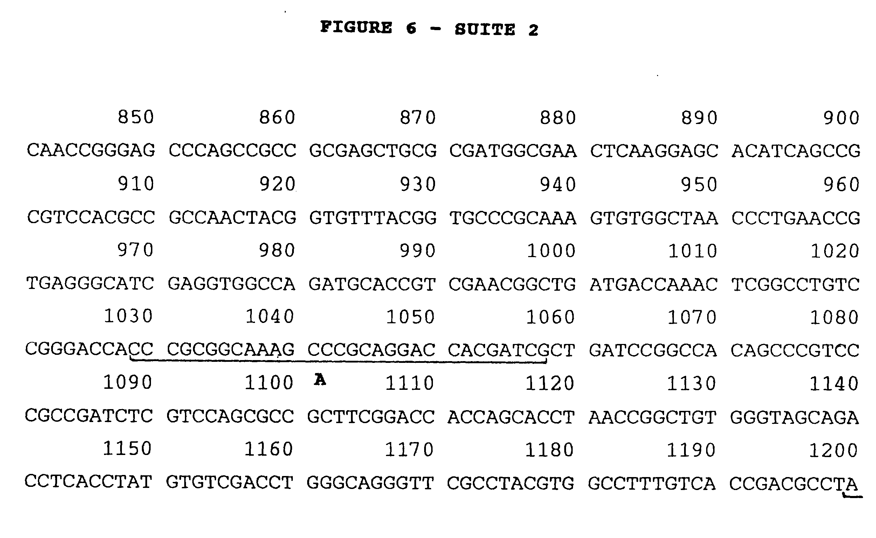

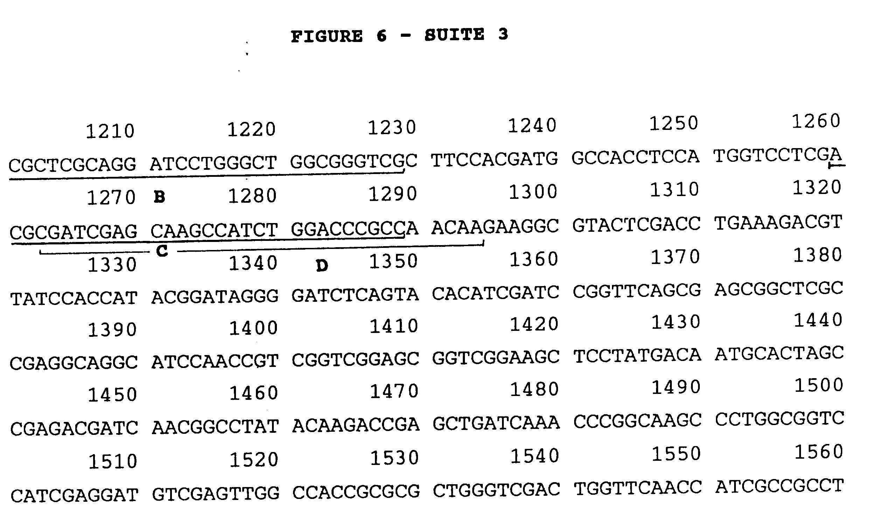

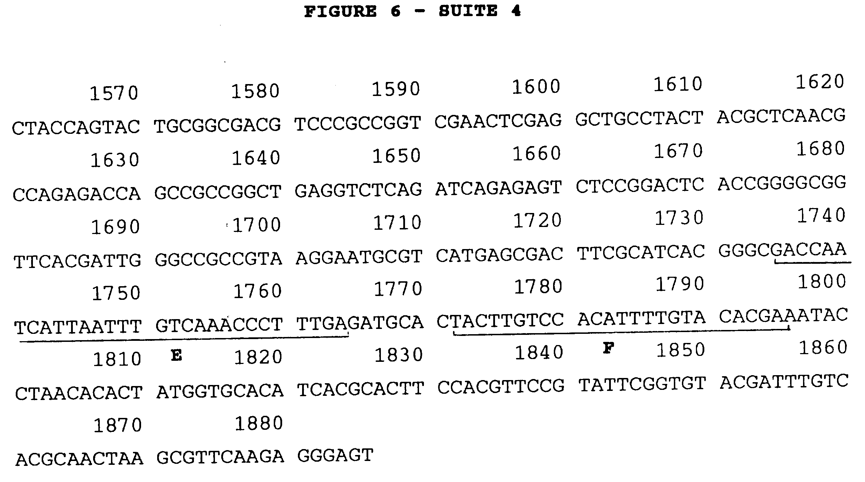

Les inventeurs ont identifié cette série de fragments d'acide nucléique apte à jouer le rôle d'amorces à partir de la séquence IS 6110 (Nucl. Acid. Res. Vol 18 n° 1, 1990) et des séquences qui jouxtent la séquence IS 6110 dans le génome du M. tuberculosis. Ces dernières ont été identifiées dans le cadre de cette invention par les inventeurs. La séquence IS 6110 décrite dans Nucl. Acid., Res., Vol. 18 n° 1, 1990, fait partie de la séquence indiquée dans la figure 6. Plus particulièrement, la séquence IS 6110 s'étend des bases 327 à 1687 de la séquence de la figure 6.

Les amorces de l'invention présentent des caractéristiques essentielles pour permettre leur utilisation dans l'amplification sélective d'ADN de M. tuberculosis, à savoir l'absence d'homologie avec le génome humain et l'absence d'amplification de séquences apparentées et susceptibles d'être présent es dans l'échantillon biologique (par exemple la séquence d'E. coli IS 3411). De plus, les inventeurs ont constaté que les résultats obtenus en utilisant les amorces de l'invention sont très fiables dans la mesure où la longueur des fragments obtenus correspond à la longueur théorique attendue et sont d'une longueur constante et non pas variable. Ceci est vrai même pour les couples d'amorces qui conduisent à l'amplification de fragments très longs (de l'ordre de 1000 à 1500 bases) où le risque d'interruption de la polymérisation est très élevé en raison des effets de la structure secondaire de la séquence.

En outre, une vérification des produits d'amplification par hybridation d'une sonde nucléique contenant la séquence indiquée dans la figure 6 ou un fragment de cette séquence confirme la fiabilité de la méthode. Ces résultats n'étaient pas prévisibles.

A partir de la séquence IS 6110, il serait possible de préparer un grand nombre d'amorces nucléiques, cependant peu d'entre elles seraient efficaces et/ou spécifiques.

La figure 6 illustre les positions des amorces A à H par rapport à la séquence entière.

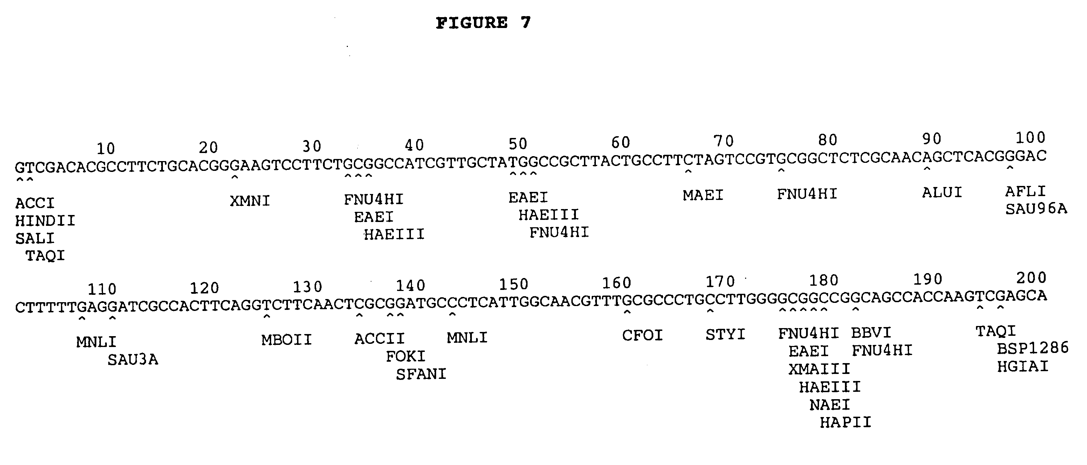

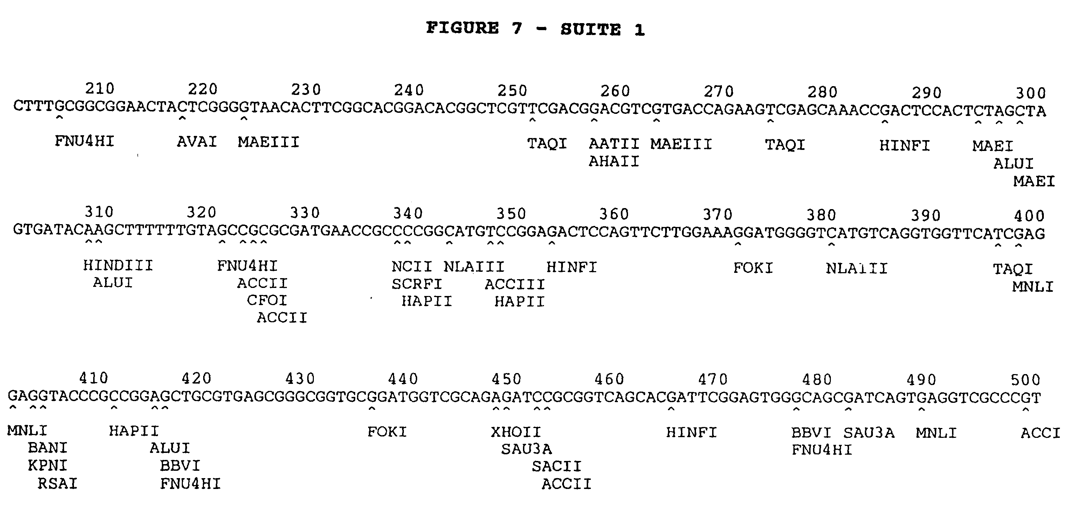

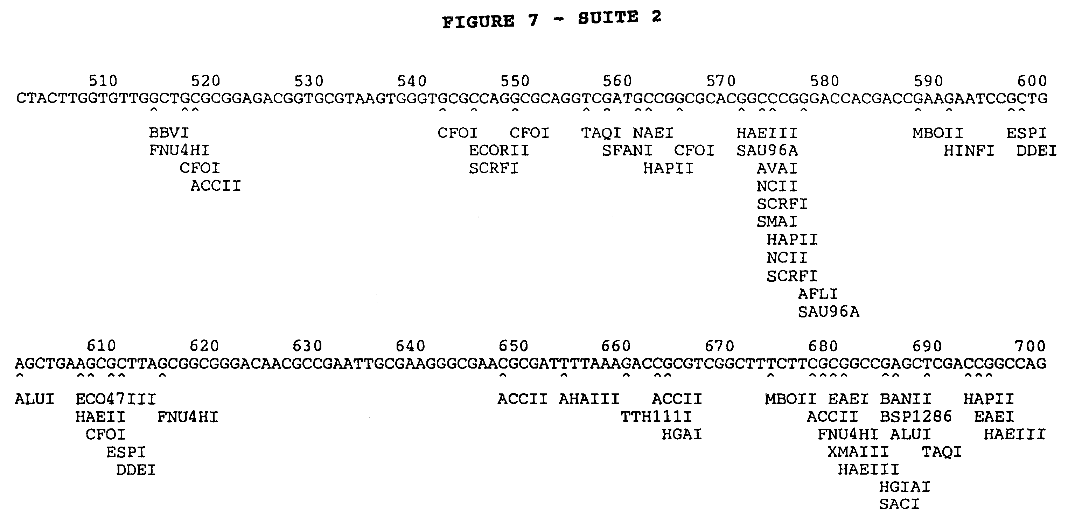

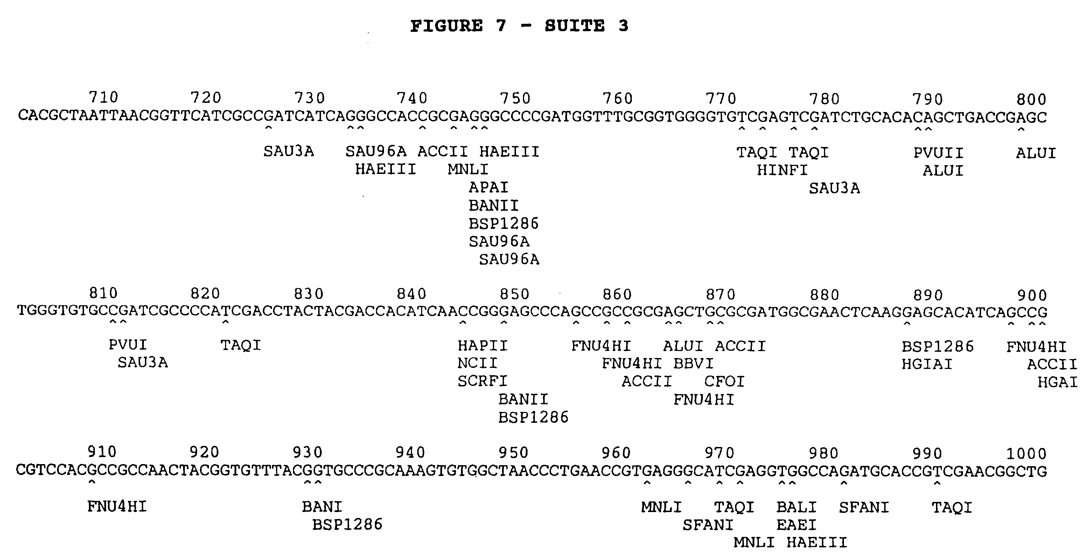

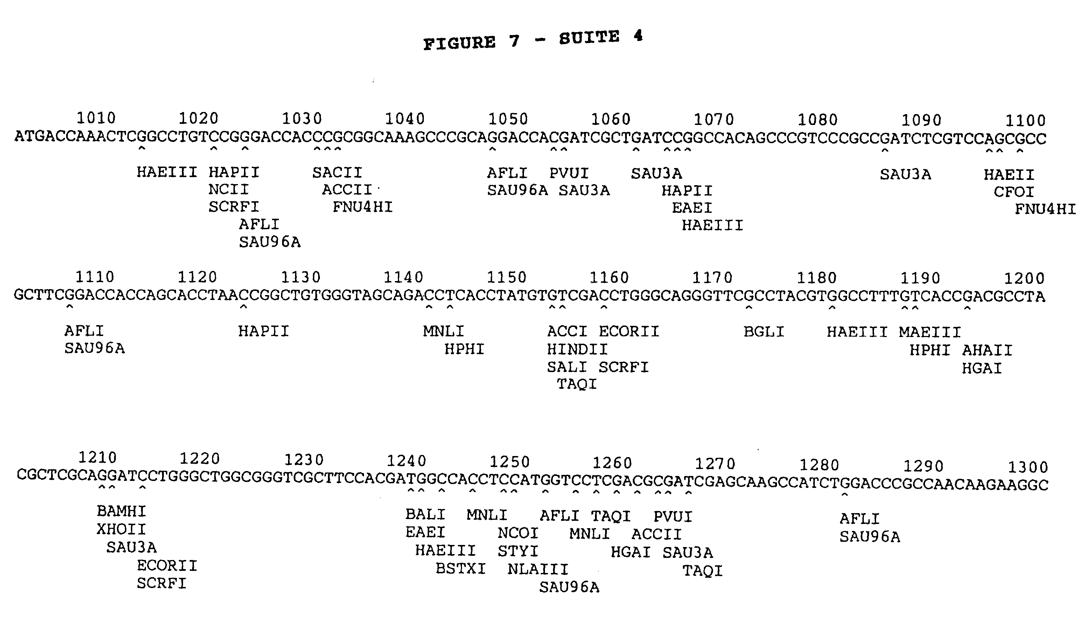

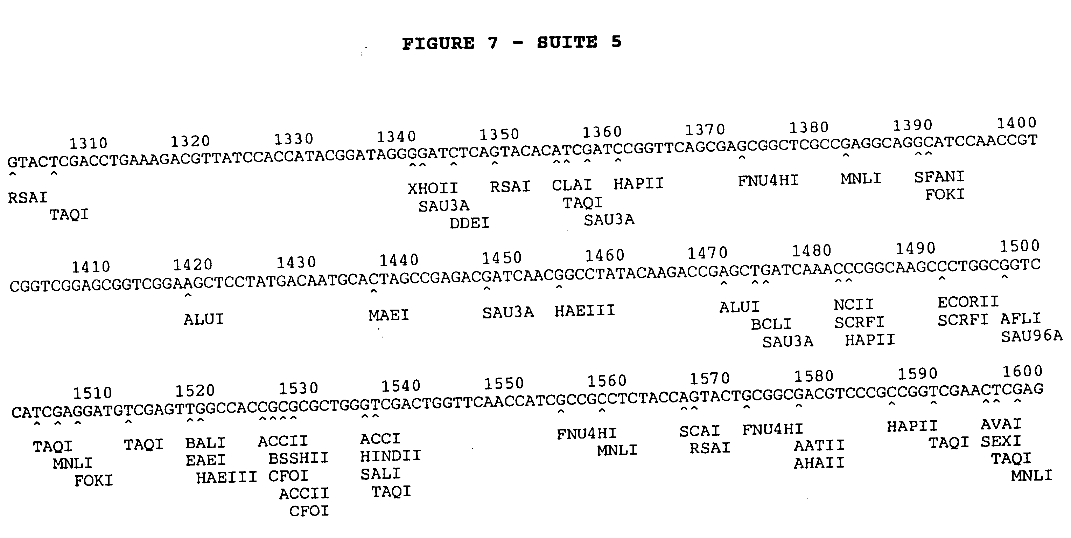

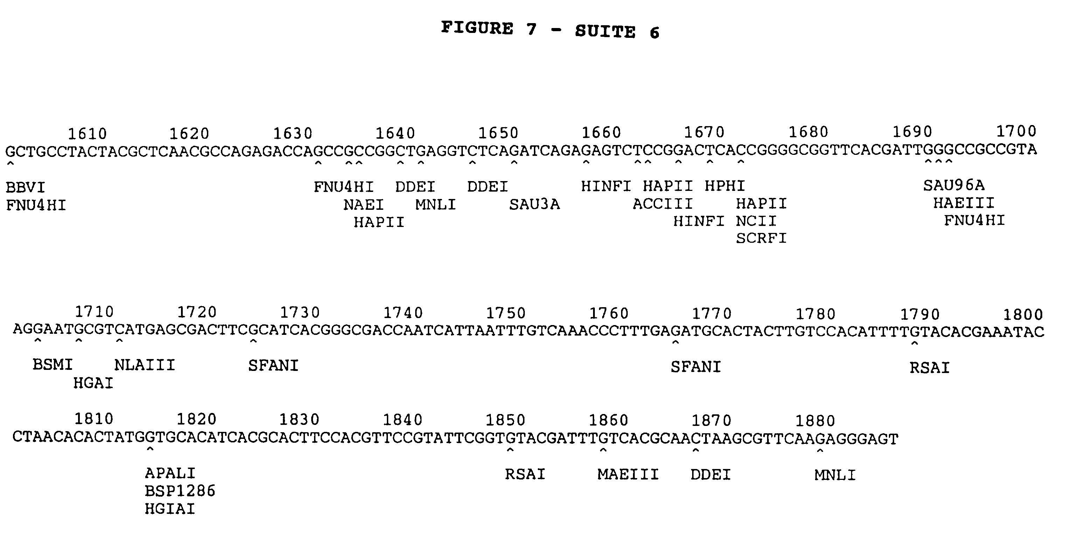

La figure 7 montre la carte de restriction de la séquence de la figure 6.

Selon un mode de réalisation de l'invention, le couple d'amorces est choisi, parmi les séquences I à IV, de façon à ce que le produit d'amplification ait une longueur entre 100 et 300 nucléotides environ, par exemple entre 100 et 200 environ. Des couples dont l'amorce positive est constituée de la séquence A et l'amorce négative est constituée de l'une des séquences B, C et D, sont particulièrement préférés. Un autre couple d'amorces particulièrement préféré est celui dont l'amorce positive est constituée de la séquence H et l'amorce négative est constituée de la séquence complémentaire à la séquence G.

Les amorces de l'invention peuvent aussi être constituées d'une séquence II qui a une longueur de 20 à 40 bases et qui comporte au moins 10 bases consécutives de l'une des séquences A à H. Comme exemple de ce type d'amorce, on peut citer des fragments de l'une des séquences A à H ayant entre 20 et 30 hases, ou encore, l'une des séquences A à H à laquelle ont été rajoutés des linkers, par exemple un linker EcoRI, GAAT. Il est particulièrement préféré d'utiliser des amorces dont les 5 premiers nucléotides côté 3' sont 100 % homologues à ceux présents dans la partie correspondante de la séquence à amplifier.

Il est également possible d'utiliser comme amorce une séquence III ayant une longueur de 20 à 40 bases qui hybride dans des conditions stringentes avec la séquence I ou II. Ce type de séquence présente en général au moins 80 % d'homologie avec la séquence à laquelle elle s'hybride. De cette manière, il est possible de substituer certaines bases des séquences A à H avec d'autres bases ou de rajouter des bases aux extrémités des séquences A à H. Les conditions stringentes sont celles normalement utilisées dans l'art.

L'invention concerne aussi des séquences IV qui sont des séquences complémentaires à l'une des séquences I, II ou III, par exemple complémentaires à l'une des séquences A à H.

L'invention concerne également une méthode de détection de la présence de Mycobacterium tuberculosis dans un échantillon biologique, caractérisée par les étapes suivantes :
i) mise en contact de l'échantillon biologique avec un couple de fragments d'acide nucléique, dits amorces, selon l'invention, l'ADN contenu dans l'échantillon ayant été, le cas échéant, préalablement rendu accessible à l'hybridation et dans des conditions permettant une hybridation des amorces à l'ADN de Mycobacterium tuberculosis ;
ii) amplification de l'ADN de Mycobacterium tuberculosis ;
iii) mise en évidence de l'amplification de fragments d'ADN correspondant au fragment encadré par les amorces, par exemple par électrophorèse sur gel ;
iv) vérification éventuelle de la séquence du fragment amplifié, par exemple par hybridation de sonde spécifique, par séquençage ou par analyse de site de restriction.

L'échantillon biologique peut être n'importe quel échantillon susceptible de contenir du M. tuberculosis, par exemple des crachats, de l'urine, du sang. Normalement, les échantillons subissent un traitement afin d'extraire l'ADN et de le rendre accessible à l'hybridation. Ces traitements sont connus dans l'art.

Les conditions appliquées lors de l'amplification peuvent être les suivantes :

| | | | |
|---|---|---|---|
| 1er cycle : | i) environ 94°C | 5 minutes | )1 X ) |
| | ii) environ 60°C | 1 minute | |
| | | | |
| cycles | i) environ 94°C | 15 secondes | )20 à )40 X |
| suivants : | ii) environ 60°C | 1 minute | |
| | | | |
| dernier | i) environ 94°C | 15 secondes | )1 X ) |
| cycle : | ii) environ 60°C | 5 minutes ) | |

La mise en évidence de l'amplification peut être effectuée par électrophorèse sur gel, par exemple sur gel d'agarose coloré au bromure d'éthidium. Après avoir effectué l'amplification, il est possible, dans le cadre de l'invention, de vérifier la séquence du fragment amplifié par exemple par hybridation de sonde nucléique, ladite sonde comprenant au moins une partie de la séquence. De telles sondes sont des plasmides pMT01, contenant les bases 1 à 1152 de la séquence de la figure 6 et le plasmide pMT02, contenant les bases 309 à 1219 de ladite séquence. D'autres sondes appropriées seraient toute sonde ayant une longueur d'environ 20 à 300 bases, capable de s'hybrider dans des conditions stringentes avec une partie de la séquence IS 6110 se trouvant entre les deux amorces choisies. Des sondes particulièrement préférées sont les séquences J, K, L, M suivantes :

J : 5' - CTGATCCGGCCACAGCCCGTCCCGCCGATC - 3'
K : 5' - AGGCGTCGGTGACAAAGGCCACGTAGGCGA - 3'
L : 5' - CGAGGACCATGGAGGTGGCCATCGTGGAAG - 3'
M : 5' - TGCCCTCATTGGCAACGTTTGCGCCCTGCC - 3'

Les conditions d'hybridation appliquées lors d'une telle vérification pourraient être les suivantes :

| | | |
|---|---|---|
| hybridation : | environ 65 à 68°C | - 6 x SSC 10 % dextran sulfate 5 x Denhardt's 10 mM EDTA 0.5 % SDA 100 µg/ml d'ADN de sperme de saumon |
| lavage : | environ 65°C | -2 x SSC (deux FOIS 10 min) 2xSSc+0.1% SDS (une fois 30 mn) 0.1 x SSC (une fois 10 min) |

1 x SSC correspond à 0.15 M NaCl et 0.05 M citrate de Na et une solution 1 x Denhardt's correspond à 0.02 % Ficoll, 0.02 % de polyvinylpyrrolidone et 0.02 % de sérum albumine bovine.

D'autres moyens de vérifier les produits d'amplification consiste en le séquençage direct du fragment ou une analyse par site de restriction. Toutefois, cette vérification n'est pas une étape obligatoire de la méthode, les amorces de l'invention conduisant à une amplification très fidèle de la séquence.

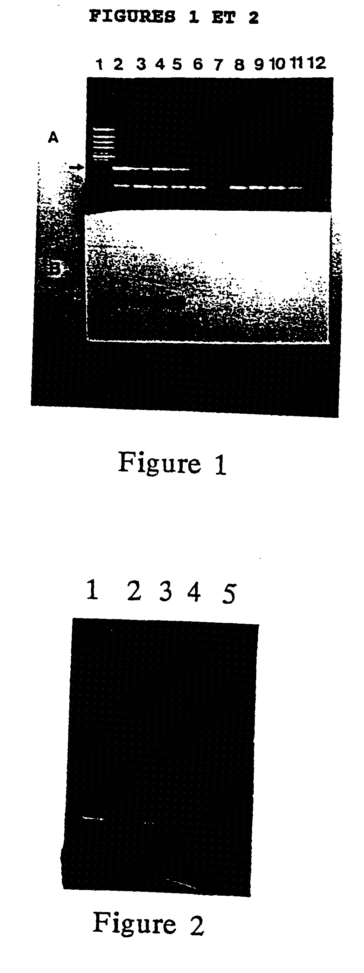

Il est à noter que l'amplification selon l'invention est spécifique de l'ADN du complexe Mycobacterium tuberculosis (voir par exemple figures 1A et B). L'amplification observée avec l'ADN de M. bovis-BCG, M. bovis et M. microti ne diminue pas l'intérêt de la méthode dans la mesure où ces mycobactéries ne sont pas susceptibles d'être présentes dans l'échantillon d'origine humaine. M. Bovis est responsable de la tuberculose chez les bovins et M. Microti est l'agent causal de la tuberculose des rongeurs. Les amorces de l'invention ne conduisent à aucune amplification d'ADN issu d'autres types de Mycobacteria tels que M. fortiutum, M. gordonae, M. avium, etc...

En outre, les amorces de l'invention n'amplifient pas d'ADN d'origine humaine ou bactérienne (par exemple E. Coli). Ceci est illustré dans la figure 2.

L'invention concerne également un kit ou nécessaire pour la détection de la présence de Mycobacterium tuberculosis dans un échantillon biologique, caractérisé en ce qu'il comporte les éléments suivants :
- un couple de fragments d'acide nucléique selon l'une quelconque des revendications 1 à 5 ;
- les réactifs nécessaires pour effectuer une amplification d'ADN ;
- éventuellement un composant permettant de vérifier la séquence du fragment amplifié, plus particulièrement une sonde nucléique telle que définie précédemment.

L'invention concerne en outre la séquence entière illustrée dans la figure 6. Les inventeurs ont constaté que cette séquence contient deux cadres ouverts de lecture dont un ressemble à un gène codant pour un transposase.

L'invention sera illustrée par les exemples non-limitatifs suivants.

### EXEMPLES

### Exemple 1 : sélection et synthèse des couples d'amorces oligonucléotidiques

A partir de la séquence complète illustrée dans la figure 6, plusieurs couples d'amorces oligonucléotidiques ont été sélectionnés et synthétisés. Ces couples d'amorces sont illustrés ci-dessous. Pour certains de ces couples d'amorces, les séquences des sondes oligonucléotidiques susceptibles d'être utilisées pour détecter les produits d'amplification sont indiquées :
couple d'amorces n°1 
amorce positive : 5' - CCCGCGGCAAAGCCCGCAGGACCACGATCG - 3'

amorce négative : 5' - CGACCCGCCAGCCCAGGATCCTGCGAGCGT - 3'
 longueur du fragment amplifié hors amorces : 141
   sondes du couple n°1 
1) 5' - CTGATCCGGCCACAGCCCGTCCCGCCGATC - 3'

2) 5' - AGGCGTCGGTGACAAAGGCCACGTAGGCGA - 3'
couple d'amorces n°2 
amorce positive : 5' - CCCGCGGCAAAGCCCGCAGGACCACGATCG - 3'
amorce négative : 5' - GGCGGGTCCAGATGGCTTGCTCGATCGCGT - 3'
 longueur du fragment amplifié hors amorces: 201
   sondes du couple n°2 
1) 5' - CTGATCCGGCCACAGCCCGTCCCGCCGATC - 3'

2) 5' - CGAGGACCATGGAGGTGGCCATCGTGGAAG - 3'
couple d'amorces n°3 
amorce positive : 5' - CCCGCGGCAAAGCCCGCAGGACCACGATCG - 3'
amorce négative : 5' - GTTGGCGGGTCCAGATGGCTTGCTCGATCG - 3
 longueur du fragment amplifié hors amorces: 204
   sondes du couple n°3 
1) 5' - CTGATCCGGCCACAGCCCGTCCCGCCGATC - 3'

2) 5' - CGTCGAGGACCATGGAGGTGGCCATCGTGG - 3'
couple d'amorces n°4 
amorce positive : 5'-CCCGCGGCAAAGCCCGCAGGACCACGATCG-3'

amorce négative : 5'-        TCAAAGGGTTTGACAAATTAATGATTGGTC-3'
 longueur du fragment amplifié hors amorce : 740
couple d'amorces n°5 
amorce positive : 5'-CCCGCGGCAAAGCCCGCAGGACCACGATCG-3'
amorce négative : 5'-        TCGTGTACAAAATGTGGACAAGTA-3'
 longueur du fragment amplifié hors amorce : 770
couple d'amorces n°6 
amorce positive : 5'- [?]TCGACGGACGTCGTGACCAGAAGTC-3'
amorce négative : 5' - CGACCCGCCAGCCCAGGATCCTGCGAGCGT - 3'
 longueur du fragment amplifié hors amorce : 980
couple d'amorces n°7 
amorce positive : 5'- TCGACGGACGTCGTGACCAGAAGTC-3'
amorce négative : 5'- GGCGGGTCCAGATGGCTTGCTCGATCGCGT-3'
 longueur du fragment amplifié hors amorce : 1040
couple d'amorces n°8 
amorce positive : 5'- TCGACGGACGTCGTGACCAGAAGTC-3'
amorce négative : 5'- TCGTGTACAAAATGTGGACAAGTA-3'
 longueur du fragment amplifié hors amorce : 1550
couple d'amorces n° 9 
amorce positive :    5'-GTCGACACGCCTTCTGCACG GGAAGTCCTT-3'
amorce négative : 5'-GACTTCTGGTCACGACGTCCGTCGAA-3'
 longueur du fragment amplifié hors amorces : 219
   sonde du couple n°9

5'-TGCCCTCATTGGCAACGTTTGCGCCCTGCC-3'

### Exemple 2 : vérification de la spécificité des amorces par rapport à d'autres types de Mycobacteries.

La spécificité des amorces a été vérifiée en utilisant de l'ADN de différentes espèces bactériennes appartenant au genre Mycobacterium.

L'ADN total provenant d'échantillons de différents types de Mycobacteria est soumis à une amplification par la technique de "Polymerase Chain Reaction" (P.C.R.) en utilisant le couple d'amorces n° 1 indiqué dans l'exemple 1.

Les paramètres des étapes de P.C.R. ont été choisis de la façon suivante :

| | | | |
|---|---|---|---|
| 1er cycle : | i) 94°C | 5 minutes | )1 X ) |
| | ii) 60°C | 1 minute | |
| | | | |
| cycles | i) 94°C | 15 secondes | )20 à )40 X |
| suivants : | ii) 60°C | 1 minute | |
| | | | |
| dernier | i) 94°C | 15 secondes | )1 X ) |
| cycle : | ii) 60°C | 5 minutes | |

Les produits d'amplification sont analysés par électrophorèse en gel d'agarose et coloration au bromure d'éthidium.

La figure 1A montre les résultats. Les voies indiquées dans la figure 1A correspondent aux échantillons suivants :

| | |
|---|---|
| 1- Marqueurs de taille | 7- M. gordonae |
| 2 - Mycobacterium tuberculosis | 8- M. intracellularae |
| 3- M. bovis-BCG | 9- M. paratuberculosis |
| 4- M. bovis | 10- M. scrofulaceum |
| 5- M. microti | 11- M. avium |
| 6- M. fortiutum | 12- tampon TE |

La figure 1B montre les résultats obtenus lorsque le plasmide pMT02 (marqué par l'AAF selon Kourilsky et al, demande de brevet français 8124131) a été utilisé comme sonde sur les produits d'amplification obtenus dans cet exemple. La construction du plasmide pMT02 est décrit dans l'exemple 6.

### Exemple 3 : vérification de la spécificté des amorces par rapport à de l'ADN provenant d'Escherichia Coli ou de cellules humaines

L'ADN humain peut contaminer les échantillons à analyse:r. La technique d'amplification décrite dans l'exemple 2 est appliquée à des échantillons d'ADN total en présence du couple d'amorce n° 1.

Les produits d'amplifications sont analysés par électrophorèse en gel d'agarose et coloration au bromure d'éthidium. La figure 2 montre les résultats, les différentes voies correspondant aux échantillons suivants :
1- Mycobacterium tuberculosis
2- ADN humain
3- Mycobacterium tuberculosis + ADN humain
4- ADN d'Escherichia coli
5- tampon TE

### Exemple 4 : utilisation des amorces sur des ADN d'échantillons biologiques

10 µl des échantillons amplifiés provenant d'expectoration de malades de la tuberculose sont déposés sur un gel d'agarose à 2 % dans un tampon TAE (0.04M Tris-acétate, 0.001M EDTA) et 1 µg/ml EtBr.

L'amplification est réalisée par la technique de polymerase chain reaction (P.C.R.) selon Saiki et al (Science, 1988, 239, 487-491) en utilisant 12.5 pmoles des oligonucléotides (couple d'amorces n° 1) et l'ADN d'échantillons biologiques avec 2 U de Taq polymérase dans un tampon 50mM KC1, 10mM Tris-HC1 pH8.3, 2.4mM MgC1₂, 300 µM de désoxyribonucléotides et 100 µg/ml de gélatine. Le volume final de la réaction est de 100 µl. Les paramètres des étapes de P.C.R. ont été choisis de la façon suivante : 1 mn à 94°C, 1 mn à 50°C, 1 mn à 72°C ceci pendant 40 cycles.

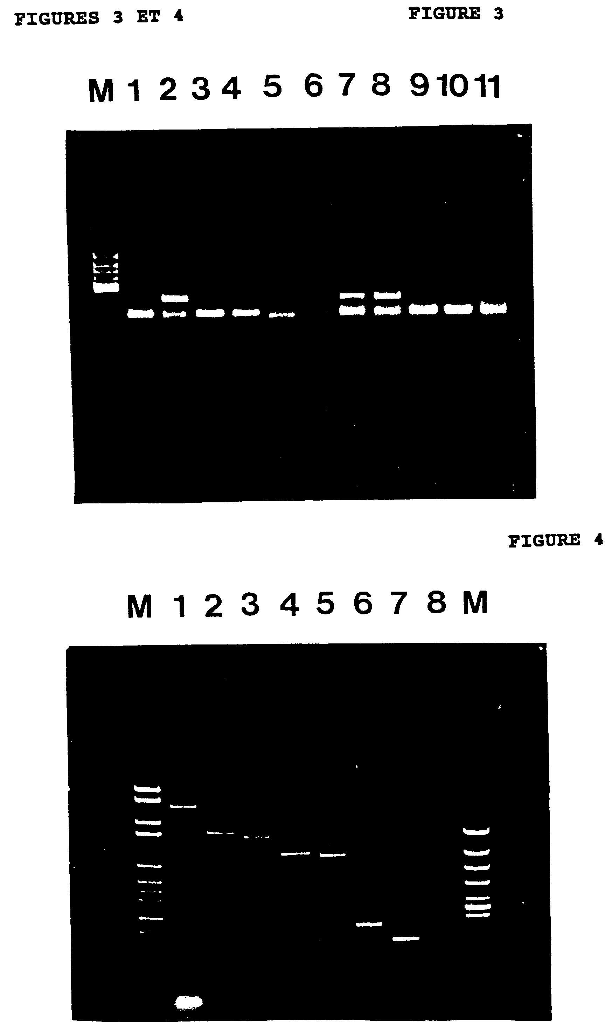

La figure 3 montre les résultats de l'analyse sur gel d'agarose après P.C.R. de ces échantillons. Les voies 1 à 11 correspondent à des échantillons biologiques provenant de 11 personnes différentes. Ces résultats étaient vérifiés par lecture directe au microscope et confirmaient les résultats obtenus par amplification:
échantillons biologiques négatifs en lecture directe : lignes 1-3-4-5-6-9-10 ;
échantillons biologiques positifs en lecture directe : lignes 2-7-8-11 ;
les bandes amplifiées sont visualisées sous UV.

### Exemple 5 : analyse sur gel d'agarose d'ADN M. tuberculosis amplifié avec différents couples d'oligonucléotides.

10 µl des échantillons amplifiés sont déposés sur un gel d' agarose à 2 %. L'amplification est réalisée selon la technique déjà décrite en utilisant plusieurs couples d'amorces décrits dans l'exemple 1. La figure 4 montre ces résultats :
ligne 1 : couple d'amorce n° 8
ligne 2 : couple d'amorce n° 7
ligne 3 : couple d'amorce n° 6
ligne 4 : couple d'amorce n° 5
ligne 5 : couple d'amorce n° 4
ligne 6 : couple d'amorce n° 2
ligne 7 : couple d'amorce n° 1
ligne 8 : contrôle négatif
M = marqueur

Les bandes amplifiées sont visualisées sous UV.

Ces résultats confirment que les fragments amplifiés sont d'une longueur correspondant à la longueur théorique, calculée à partir de la distance entre chaque amorce. Il est surprenant que malgré l'utilisation de certains couples d'amorces conduisant à l'amplification de fragments très longs, aucune interruption de la polymérisation résultant d'une structure secondaire de la séquence n'est observée.

Les résultats étaient vérifiés par hybridation avec le plasmide pMTO1 (CNCM I-900 déposé le 25/8/89) qui contient les bases 1 à 1152 de la séquence illustrée dans la figure 6.

### Exemple 6 : construction du plasmide pMTO2

Le plasmide pMT02 a été construit par clonage dans le vecteur pUC18 d'un fragment de 900 paires de bases Hind III/Bam HI issus de la séquence IS 6110 (fragment qui correspond aux bases 309 à 1219 de la séquence illustrée dans la figure 6).

Le plasmide pMT02 peut servir de sonde lors de la vérification des séquences amplifiées. La spécificité de pMT02 a été déterminée par Southern blot après digestion complète de différents ADN mycobactériens par Bam HI.

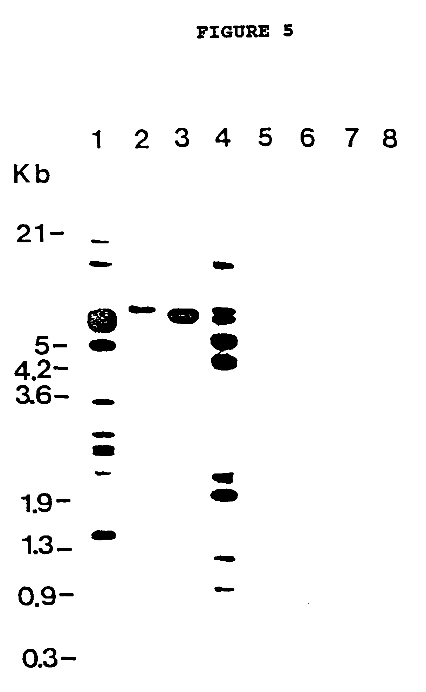

Les résultats sont indiqués dans la figure 5.

Les différentes voies de la figure 5 ont les significations suivantes :

| | |
|---|---|
| 1- M. tuberculosis | ) |
| 2- M. bovis-BCG | ) complexe |
| 3- M. bovis | ) tuberculosis |
| 4- M. microti | ) |
| | |
| 5- M. paratuberculosis | ) |
| 6- M. intracellularae | ) complexe |
| 7- M. scrofulaceum | ) avium |
| 8- M. avium | ) |

## Revendications

1. Fragment d'acide nucléique dérivé du génome de Mycobacterium tuberculosis caractérisé en ce qu'il est constitué d'une des séquences I, II, III et IV, définie de la façon suivante :
I : une séquence choisie parmi l'une des séquences A à H : 
A : 5' - CCCGCGGCAAAGCCCGCAGGACCACGATCG - 3'
B : 5' - CGACCCGCCAGCCCAGGATCCTGCGAGCGT - 3'
C : 5' - GGCGGGTCCAGATGGCTTGCTCGATCGCGT - 3'
D : 5' - GTTGGCGGGTCCAGATGGCTTGCTCGATCG - 3'
E : 5' - TCAAAGGGTTTGACAAATTAATGATTGGTC - 3'
F : 5' - TCGTGTACAAAATGTGGACAAGTA - 3'
G : 5' - TCGACGGACGTCGTGACCAGAAGTC - 3'
H : 5' - GTCGACACGCCTTCTGCACGGGAAGTCCTT - 3'
II : une séquence comportant au moins 10 bases consécutives de l'une des séquences A à H et ayant une longueur totale d'environ 20 à 40 bases ;
III : une séquence ayant une longueur de 20 à 40 bases qui hybride avec la séquence I ou avec la séquence II, et qui présente de préférence au moins 80% d'homologie avec celles-ci ;
IV : une séquence complémentaire à l'une des séquences I, II ou III.

2. Couple de fragments d'acide nucléique dérivés du génome de Mycobacterium tuberculosis et aptes à jouer le rôle d'amorces nucléiques dans l'amplification de l'ADN provenant dudit Mycobacterium dans un échantillon biologique, caractérisé en ce qu'il est constitué de deux séquences sélectionnées parmi les séquences I à IV selon la revendication 1.

3. Couple de fragments selon la revendication 2 caractérisé en ce qu'au moins un des membres du couple est constitué par une séquence appartenant au groupe I.

4. Couple de fragments selon la revendication 2 ou 3, caractérisé en ce qu'il est constitué d'une part de l'une des séquences A ou G, et, d'autre part, de l'une des séquences B, C, D, E, F ou encore de la séquence H et la séquence complémentaire à la séquence G.

5. Couple de fragments selon la revendication 4 caractérisé en ce qu'il est constitué d'une part de la séquence A et, d'autre part, de l'une des séquences B, C, D.

6. Méthode de détection de la présence de Mycobacterium tuberculosis dans un échantillon biologique, caractérisée par les étapes suivantes :
i) mise en contact de l'échantillon biologique avec un couple de fragments d'acide nucléique, dits amorces, selon l'une quelconque des revendications 1 à 5, l'ADN contenu dans l'échantillon ayant été, le cas échéant, préalablement rendu accessible à l'hybridation et dans des conditions permettant une hybridation des amorces à l'ADN de Mycobacterium tuberculosis ;
ii) amplification de l'ADN de Mycobacterium tuberculosis ;
iii) mise en évidence de l'amplification de fragments d'ADN correspondant au fragment encadré par les amorces, par exemple par électrophorèse sur gel ;
iv) vérification éventuelle de la séquence du fragment amplifié, par exemple par hybridation de sonde spécifique, par séquençage ou par analyse de site de restriction.

7. Méthode de détection selon la revendication 6 caractérisée en ce que les conditions appliquées lors de l'amplification de l'ADN sont les suivantes :
| | | | |
|---|---|---|---|
| 1er cycle : | i) environ 94°C | 5 minutes | ) 1 X |
| | ii) environ 60°C | 1 minute ) | ) |
| | | | |
| cycles | i) environ 94°C | 15 secondes | ) 20 à |
| suivants : | ii) environ 60°C | 1 minute | ) 40 X |
| | | | |
| dernier | i) environ 94°C | 15 secondes | ) 1 X |
| cycle : | ii) environ 60°C | 5 minutes | ) |

8. Sonde nucléique apte à vérifier les produits d'amplification résultant du procédé de détection selon l'une quelconque des revendications 6 ou 7, caractérisée en ce qu'elle comporte :
i) soit une séquence ayant une longueur d'environ 20 à 300 bases capable de s'hybrider avec une partie de la séquence IS 6110 se situant entre les deux amorces nucléiques ;
ii) le plasmide pMTO2, créé par l'insertion du fragment HindIII-BamHI, correspondant aux bases 309 à 1219 de la séquence de la figure 6, dans le plasmide pUC18.

9. Sonde nucléique selon la revendication 8 caractérisée en ce qu'elle a une longueur comprise entre 20 et 300 bases et comporte au mpins 20 bases consécutives de l'une des séquences J, K, L ou M suivantes :

J : 5' - CTGATCCGGCCACAGCCCGTCCCGCCGATC - 3'
K : 5' - AGGCGTCGGTGACAAAGGCCACGTAGGCGA - 3'
L : 5' - CGAGGACCATGGAGGTGGCCATCGTGGAAG - 3'
M : 5' - TGCCCTCATTGGCAACGTTTGCGCCCTGCC - 3'

10. Kit ou nécessaire pour la détection de la présence de Mycobacterium tuberculosis dans un échantillon biologique, caractérisé en ce qu'il comporte les éléments suivants :
- un couple de fragments d'acide nucléique selon l'une quelconque des revendications 1 à 5 ;
- les réactifs nécessaires pour effectuer une amplification d'ADN ;
- éventuellement un composant permettant de vérifier la séquence du fragment amplifié, plus particulièrement une sonde nucléique selon l'une quelconque des revendications 8 ou 9.

11. Séquence d'acide nucléique, spécifique du Mycobacterium tuberculosis ayant la séquence :

10 20 30 40 50 60
GTCGACACGC CTTCTGCACG GGAAGTCCTT CTGCGGCCAT CGTTGCTATG GCCGCTTACT
70 80 90 100 110 120
GCCTTCTAGT CCGTGCGGCT CTCGCAACAG CTCACGGGAC CTTTTTGAGG ATCGCCACTT
130 140 150 160 170 180
CAGGTCTTCA ACTCGCGGAT GCCCTCATTG GCAACGTTTG CGCCCTGCCT TGGGGCGGCC
190 200 210 220 230 240
GGCAGCCACC AAGTCGAGCA CTTTGCGGCG GAACTACTCG GGGTAACACT TCGGCACGGA
250 260 270 280 290 300
CACGGCTCGT TCGACGGACG TCGTGACCAG AAGTCGAGCA AACCGACTCC ACTCTAGCTA
310 320 330 340 350 360
GTGATACAAG CTTTTTTGTA GCCGCGCGAT GAACCGCCCC GGCATGTCCG GAGACTCCAG
370 380 390 400 410 420
TTCTTGGAAA GGATGGGGTC ATGTCAGGTG GTTCATCGAG GAGGTACCCG CCGGAGCTGC
430 440 450 460 470 480
GTGAGCGGGC GGTGCGGATG GTCGCAGAGA TCCGCGGTCA GCACGATTCG GAGTGGGCAG
490 500 510 520 530 540
CGATCAGTGA GGTCGCCCGT CTACTTGGTG TTGGCTGCGC GGAGACGGTG CGTAAGTGGG
550 560 570 580 590 600
TGCGCCAGGC GCAGGTCGAT GCCGGCGCAC GGCCCGGGAC CACGACCGAA GAATCCGCTG
610 620 630 640 650 660
AGCTGAAGCG CTTAGCGGCG GGACAACGCC GAATTGCGAA GGGCGAACGC GATTTTAAAG
670 680 690 700 710 720
ACCGCGTCGG CTTTCTTCGC GGCCGAGCTC GACCGGCCAG CACGCTAATT AACGGTTCAT
730 740 750 760 770 780
CGCCGATCAT CAGGGCCACC GCGAGGGCCC CGATGGTTTG CGGTGGGGTG TCGAGTCGAT
790 800 810 820 830 840
CTGCACACAG CTGACCGAGC TGGGTGTGCC GATCGCCCCA TCGACCTACT ACGACCACAT
850 860 870 880 890 900
CAACCGGGAG CCCAGCCGCC GCGAGCTGCG CGATGGCGAA CTCAAGGAGC ACATCAGCCG

910 920 930 940 950 960
CGTCCACGCC GCCAACTACG GTGTTTACGG TGCCCGCAAA GTGTGGCTAA CCCTGAACCG
970 980 990 1000 1010 1020
TGAGGGCATC GAGGTGGCCA GATGCACCGT CGAACGGCTG ATGACCAAAC TCGGCCTGTC
1030 1040 1050 1060 1070 1080
CGGGACCACC CGCGGCAAAG CCCGCAGGAC CACGATCGCT GATCCGGCCA CAGCCCGTCC
1090 1100 1110 1120 1130 1140
CGCCGATCTC GTCCAGCGCC GCTTCGGACC ACCAGCACCT AACCGGCTGT GGGTAGCAGA
1150 1160 1170 1180 1190 1200
CCTCACCTAT GTGTCGACCT GGGCAGGGTT CGCCTACGTG GCCTTTGTCA CCGACGCCTA
1210 1220 1230 1240 1250 1260
CGCTCGCAGG ATCCTGGGCT GGCGGGTCGC TTCCACGATG GCCACCTCCA TGGTCCTCGA
1270 1280 1290 1300 1310 1320
CGCGATCGAG CAAGCCATCT GGACCCGCCA ACAAGAAGGC GTACTCGACC TGAAAGACGT
1330 1340 1350 1360 1370 1380
TATCCACCAT ACGGATAGGG GATCTCAGTA CACATCGATC CGGTTCAGCG AGCGGCTCGC
1390 1400 1410 1420 1430 1440
CGAGGCAGGC ATCCAACCGT CGGTCGGAGC GGTCGGAAGC TCCTATGACA ATGCACTAGC
1450 1460 1470 1480 1490 1500
CGAGACGATC AACGGCCTAT ACAAGACCGA GCTGATCAAA CCCGGCAAGC CCTGGCGGTC
1510 1520 1530 1540 1550 1560
CATCGAGGAT GTCGAGTTGG CCACCGCGCG CTGGGTCGAC TGGTTCAACC ATCGCCGCCT
1570 1580 1590 1600 1610 1620
CTACCAGTAC TGCGGCGACG TCCCGCCGGT CGAACTCGAG GCTGCCTACT ACGCTCAACG
1630 1640 1650 1660 1670 1680
CCAGAGACCA GCCGCCGGCT GAGGTCTCAG ATCAGAGAGT CTCCGGACTC ACCGGGGCGG
1690 1700 1710 1720 1730 1740
TTCACGATTG GGCCGCCGTA AGGAATGCGT CATGAGCGAC TTCGCATCAC GGGCGACCAA
1750 1760 1770 1780 1790 1800
TCATTAATTT GTCAAACCCT TTGAGATGCA CTACTTGTCC ACATTTTGTA CACGAAATAC
1810 1820 1830 1840 1850 1860
CTAACACACT ATGGTGCACA TCACGCACTT CCACGTTCCG TATTCGGTGT ACGATTTGTC
1870 1880
ACGCAACTAA GCGTTCAAGA GGGAGT

## Patentansprüche

1. Nukleinsäurefragment, das vom Genom des Mycobacterium tuberculosis abgeleitet ist, dadurch gekennzeichnet, daß es aus einer der Sequenzen I, II, III und IV besteht, die sich wie folgt definieren:
I: eine Sequenz aus den nachfolgenden Sequenzen A bis H: 
A : 5' - CCCGCGGCAAAGCCCGCAGGACCACGATCG - 3'
B : 5' - CGACCCGCCAGCCCAGGATCCTGCGAGCGT - 3'
C : 5' - GGCGGGTCCAGATGGCTTGCTCGATCGCGT - 3'
D : 5' - GTTGGCGGGTCCAGATGGCTTGCTCGATCG - 3'
E : 5' - TCAAAGGGTTTGACAAATTAATGATTGGTC - 3'
F : 5' - TCGTGTACAAAATGTGGACAAGTA - 3'
G : 5' - TCGACGGACGTCGTGACCAGAAGTC - 3'
H : 5' - GTCGACACGCCTTCTGCACGGGAAGTCCTT - 3'
II: eine Sequenz, die wenigstens 10 aufeinanderfolgende Basen aus einer der Sequenzen A bis H umfaßt und die eine Gesamtlänge von ungefähr 20 bis 40 Basen aufweist,
III: eine Sequenz mit einer Länge von 20 bis 40 Basen, die mit der Sequenz I oder mit der Sequenz II hybridisiert und die vorzugsweise mindestens 80 % Homologie mit diesen aufweist.
IV: eine Komplementärsequenz zu einer der Sequenzen I, II oder III.

2. Paar von Nukleinsäurefragmenten, die vom Genom den Mycobacterium tuberculosis abgeleitet sind und die geeignet sind, in einer biologischen Probe die Rolle von Nukleotidprimern bei der Amplifikation der DNA, die vom besagten Mycobacterium stammt, zu übernehmen, dadurch gekennzeichnet, daß es aus zwei Sequenzen besteht, die aus den Sequenzen I bis IV nach Anspruch 1 ausgewählt sind.

3. Fragmentpaar nach Anspruch 2, dadurch gekennzeichnet, daß wenigstens einer der Bestandteile des Paares aus einer Sequenz besteht, die zu der Gruppe I gehört.

4. Fragmentpaar nach Anspruch 2 oder 3, dadurch gekennzeichnet, daß es zum einen aus einer der Sequenzen A oder G und zum anderen aus einer der Sequenzen B, C, D, E, F besteht oder als weitere Möglichkeit aus der Sequenz H und aus der Komplementärsequenz zur Sequenz G besteht.

5. Fragmentpaar nach Anspruch 4, dadurch gekennzeichnet, daß es zum einen aus der Sequenz A und zum anderen aus einer der Sequenzen B, C oder D besteht.

6. Verfahren zum Nachweis des Vorhandenseins von Mycobacterium tuberculosis in einer biologischen Probe, gekennzeichnet durch die folgenden Schritte:
i) In-Kontakt-Bringen der biologischen Probe mit einem Paar von Nukleinsäurefragmenten, besagten Primern, nach einem der Ansprüche 1 bis 5, wobei die in der Probe enthaltene DNA ggf. zuvor für die Hybridisierung zugänglich gemacht wurde und in einen Zustand gebracht wurde, der die Hybridisierung der Primer an die DNA des Mycobacterium tuberculosis erlaubt,
ii) Amplifikation der DNA des Mycobacterium tuberculosis,
iii) Nachweis der Amplifikation von DNA-Fragmenten, die dem Fragment entsprechen, das von den Primern flankiert ist, z. B. durch Gelelektrophorese,
iv) eventuelle Nachprüfung der Sequenz des amplifizierten Fragments, z. B. durch Hybridisierung mit einer spezifischen Sonde, durch Sequenzierung oder durch Restriktionsanalyse.

7. Nachweisverfahren nach Anspruch 6, dadurch gekennzeichnet, daß während der Amplifikation der DNA folgende Bedingungen herrschen:

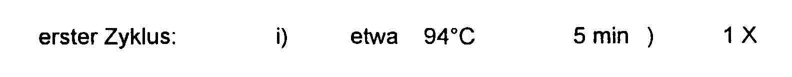

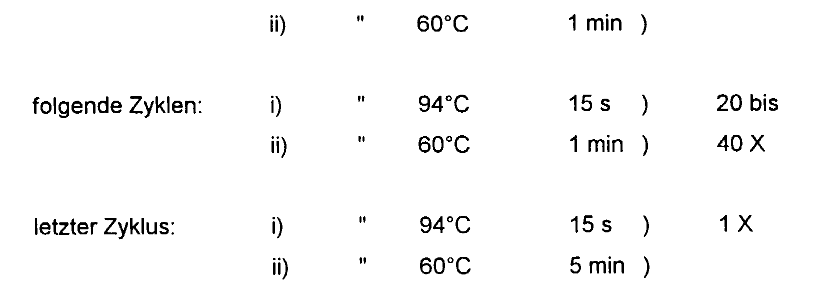

8. Gensonde, die geeignet ist, die Amplifikationsprodukte nachzuweisen, die aus einem Nachweisverfahren nach einem der Ansprüche 6 oder 7 resultieren, dadurch gekennzeichnet, daß sie umfaßt:
i) entweder eine Sequenz mit einer Länge von etwa 20 bis 300 Basen, die in der Lage ist, mit einem der Teil der Sequenz IS 6110, die sich zwischen den beiden Primern befindet, zu hybridisieren,
ii) oder das Plasmid pMT02, das durch Einsetzen des Fragments HindIII-BamHI, das den Basen 309 bis 1219 der Sequenz der Fig. 6 entspricht, in das Plasmid pUC18 entsteht.

9. Gensonde nach Anspruch 8, dadurch gekennzeichnet, daß sie eine Gesamtlänge von 20 bis 300 Basen aufweist und wenigstens 20 aufeinanderfolgende Basen einer der folgenden Sequenzen J, K, L oder M umfaßt:

J : 5' - CTGATCCGGCCACAGCCCGTCCCGCCGATC - 3'
K : 5' - AGGCGTCGGTGACAAAGGCCACGTAGGCGA - 3'
L : 5' - CGAGGACCATGGAGGTGGCCATCGTGGAAG - 3'
M : 5' - TGCCCTCATTGGCAACGTTTGCGCCCTGCC - 3'

10. Kit für den Nachweis des Vorhandenseins des Mycobacterium tuberculosis in einer biologischen Probe, dadurch gekennzeichnet, daß es folgende Elemente umfaßt:
- ein Paar von Nukleinsäurefragmenten nach einem der Ansprüche 1 bis 5,
- die notwendigen Reagenzien, um eine Amplifikation der DNA zu bewirken,
- ggf. einen Bestandteil, der die Nachprüfung der Sequenz des amplifizierten Fragments erlaubt, insbesondere eine Gensonde nach einem der Ansprüche 8 oder 9.

11. Für das Mycobacterium tuberculosis spezifische Nukleinsäuresequenz, die folgende Sequenz aufweist:

10 20 30 40 50 60
GTCGACACGC CTTCTGCACG GGAAGTCCTT CTGCGGCCAT CGTTGCTATG GCCGCTTACT
70 80 90 100 110 120
GCCTTCTAGT CCGTGCGGCT CTCGCAACAG CTCACGGGAC CTTTTTGAGG ATCGCCACTT
130 140 150 160 170 180
CAGGTCTTCA ACTCGCGGAT GCCCTCATTG GCAACGTTTG CGCCCTGCCT TGGGGCGGCC
190 200 210 220 230 240
GGCAGCCACC AAGTCGAGCA CTTTGCGGCG GAACTACTCG GGGTAACACT TCGGCACGGA
250 260 270 280 290 300
CACGGCTCGT TCGACGGACG TCGTGACCAG AAGTCGAGCA AACCGACTCC ACTCTAGCTA
310 320 330 340 350 360
GTGATACAAG CTTTTTTGTA GCCGCGCGAT GAACCGCCCC GGCATGTCCG GAGACTCCAG
370 380 390 400 410 420
TTCTTGGAAA GGATGGGGTC ATGTCAGGTG GTTCATCGAG GAGGTACCCG CCGGAGCTGC
430 440 450 460 470 480
GTGAGCGGGC GGTGCGGATG GTCGCAGAGA TCCGCGGTCA GCACGATTCG GAGTGGCAG
490 500 510 520 530 540
CGATCAGTGA GGTCGCCCGT CTACTTGGTG TTGGCTGCGC GGAGACGGTG CGTAAGTGGG
550 560 570 580 590 600
TGCGCCAGGC GCAGGTCGAT GCCGGCGCAC GGCCCGGGAC CACGACCGAA GAATCCGCTG
610 620 630 640 650 660
AGCTGAAGCG CTTAGCGGCG GGACAACGCC GAATTGCGAA GGGCGAACGC GATTTTAAAG
670 680 690 700 710 720
ACCGCGTCGG CTTTCTTCGC GGCCGAGCTC GACCGGCCAG CACGCTAATT AACGGTTCAT
730 740 750 760 770 780
CGCCGATCAT CAGGGCCACC GCGAGGGCCC CGATGGTTTG CGGTGGGGTG TCGAGTCGAT
790 800 810 820 830 840
CTGCACACAG CTGACCGAGC TGGGTGTGCC GATCGCCCCA TCGACCTACT ACGACCACAT
850 860 870 880 890 900
CAACCGGGAG CCCAGCCGCC GCGAGCTGCG CGATGGCGAA CTCAAGGAGC ACATCAGCCG

910 920 930 940 950 960
CGTCCACGCC GCCAACTACG GTGTTTACGG TGCCCGCAAA GTGTGGCTAA CCCTGAACCG
970 980 990 1000 1010 1020
TGAGGGCATC GAGGTGGCCA GATGCACCGT CGAACGGCTG ATGACCAAAC TCGGCCTGTC
1030 1040 1050 1060 1070 1080
CGGGACCACC CGCGGCAAAG CCCGCAGGAC CACGATCGCT GATCCGGCCA CAGCCCGTCC
1090 1100 1110 1120 1130 1140
CGCCGATCTC GTCCAGCGCC GCTTCGGACC ACCAGCACCT AACCGGCTGT GGGTAGCAGA
1150 1160 1170 1180 1190 1200
CCTCACCTAT GTGTCGACCT GGGCAGGGTT CGCCTACGTG GCCTTTGTCA CCGACGCCTA
1210 1220 1230 1240 1250 1260
CGCTCGCAGG ATCCTGGGCT GGCGGGTCGC TTCCACGATG GCCACCTCCA TGGTCCTCGA
1270 1280 1290 1300 1310 1320
CGCGATCGAG CAAGCCATCT GGACCCGCCA ACAAGAAGGC GTACTCGACC TGAAAGACGT
1330 1340 1350 1360 1370 1380
TATCCACCAT ACGGATAGGG GATCTCAGTA CACATCGATC CGGTTCAGCG AGCGGCTCGC
1390 1400 1410 1420 1430 1440
CGAGGCAGGC ATCCAACCGT CGGTCGGAGC GGTCGGAAGC TCCTATGACA ATGCACTAGC
1450 1460 1470 1480 1490 1500
CGAGACGATC AACGGCCTAT ACAAGACCGA GCTGATCAAA CCCGGCAAGC CCTGGCGGTC
1510 1520 1530 1540 1550 1560
CATCGAGGAT GTCGAGTTGG CCACCGCGCG CTGGGTCGAC TGGTTCAACC ATCGCCGCCT
1570 1580 1590 1600 1610 1620
CTACCAGTAC TGCGGCGACG TCCCGCCGGT CGAACTCGAG GCTGCCTACT ACGCTCAACG
1630 1640 1650 1660 1670 1680
CCAGAGACCA GCCGCCGGCT GAGGTCTCAG ATCAGAGAGT CTCCGGACTC ACCGGGGCGG
1690 1700 1710 1720 1730 1740
TTCACGATTG GGCCGCCGTA AGGAATGCGT CATGAGCGAC TTCGCATCAC GGGCGACCAA
1750 1760 1770 1780 1790 1800
TCATTAATTT GTCAAACCCT TTGAGATGCA CTACTTGTCC ACATTTTGTA CACGAAATAC
1810 1820 1830 1840 1850 1860
CTAACACACT ATGGTGCACA TCACGCACTT CCACGTTCCG TATTCGGTGT ACGATTTGTC
1870 1880
ACGCAACTAA GCGTTCAAGA GGGAGT

## Claims

1. Nucleic acid fragment derived from the Mycobacterium tuberculosis genome, characterized in that it consists of one of the sequences I, II, III and IV, defined in the following manner:
I : a sequence chosen from one of the sequences A to H: 
A : 5′ - CCCGCGGCAAAGCCCGCAGGACCACGATCG - 3′
B : 5′ - CGACCCGCCAGCCCAGGATCCTGCGAGCGT - 3′
C : 5′ - GGCGGGTCCAGATGGCTTGCTCGATCGCGT - 3′
D : 5′ - GTTGGCGGGTCCAGATGGCTTGCTCGATCG - 3′
E : 5′ - TCAAAGGGTTTGACAAATTAATGATTGGTC - 3′
F : 5′ - TCGTGTACAAAATGTGGACAAGTA - 3′
G : 5′ - TCGACGGACGTCGTGACCAGAAGTC - 3′
H : 5′ - GTCGACACGCCTTCTGCACGGGAAGTCCTT - 3′
II : a sequence containing at least 10 consecutive bases of one of the sequences A to H and having a total length of approximately 20 to 40 bases;
III : a sequence having a length of 20 to 40 bases which hybridizes with the sequence I or with the sequence II, and which preferably displays at least 80 % homology with these sequences;
IV : a sequence complementary to one of the sequences I, II and III.

2. Pair of nucleic acid fragments derived from the Mycobacterium tuberculosis genome and capable of playing the part of nucleic acid primers in the amplification of the DNA originating from the said Mycobacterium in a biological sample, characterized in that it consists of two sequences selected from the sequences I to IV according to Claim 1.

3. Pair of fragments according to Claim 2, characterized in that at least one of the members of the pair consists of a sequence belonging to the group I.

4. Pair of fragments according to Claim 2 or 3, characterized in that it consists, on the one hand of one of the sequences A and G, and on the other hand of one of the sequences B, C, D, E and F, or alternatively of the sequence H and the sequence complementary to the sequence G.

5. Pair of fragments according to Claim 4, characterized in that it consists, on the one hand of the sequence A, and on the other hand of one of the sequences B, C and D.

6. Method of detecting the presence of Mycobacterium tuberculosis in a biological sample, characterized by the following steps:
i) bringing the biological sample into contact with a pair of nucleic acid fragments, termed primers, according to any one of Claims 1 to 5, the DNA contained in the sample having previously been, where appropriate, rendered accessible to hybridization and under conditions permitting a hybridization of the primers to Mycobacterium tuberculosis DNA;
ii) amplification of the Mycobacterium tuberculosis DNA;
iii) demonstration of the amplification of DNA fragments corresponding to the fragment flanked by the primers, for example by gel electrophoresis;
iv) verification, where appropriate, of the sequence of the amplified fragment, for example by specific probe hybridization, by sequencing or by restriction site analysis.

7. Detection method according to Claim 6, characterized in that the conditions applied during the amplification of the DNA are the following:

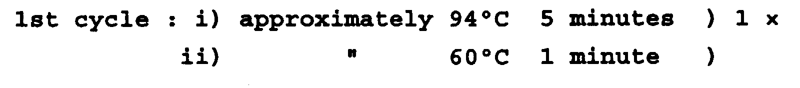

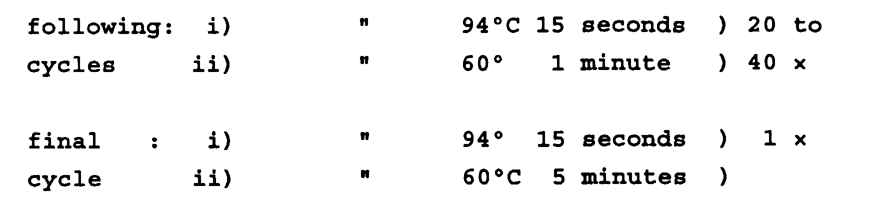

8. Nucleic acid probe capable of verifying the amplification products resulting from the detection method according to either of Claims 6 and 7, characterized in that it contains:
i) either a sequence having a length of approximately 20 to 300 bases, capable of hybridizing with a portion of the sequence IS 6110 located between the two nucleic acid primers;
ii) plasmid pMT02, created by inserting the HindIII-BamHI fragment corresponding to bases 309 to 1219 of the sequence of Figure 6 into plasmid pUC18.

9. Nucleic acid probe according to Claim 8, characterized in that it has a length of between 20 and 300 bases and contains at least 20 consecutive bases of one of the following sequences J, K, L and M:

J : 5' - CTGATCCGGCCACAGCCCGTCCCGCCGATC - 3'
K : 5' - AGGCGTCGGTGACAAAGGCCACGTAGGCGA - 3'
L : 5' - CGAGGACCATGGAGGTGGCCATCGTGGAAG - 3'
M : 5' - TGCCCTCATTGGCAACGTTTGCGCCCTGCC - 3'

10. Kit or outfit for detection of the presence of Mycobacterium tuberculosis in a biological sample, characterized in that it contains the following items:
- a pair of nucleic acid fragments according to any one of Claims 1 to 5;
- the reactants needed for performing a DNA amplification;
- where appropriate, a component enabling the sequence of the amplified fragment to be verified, more especially a nucleic acid probe according to either of Claims 8 and 9.

11. Nucleic acid sequence specific to Mvcobacterium tuberculosis, having the sequence:
